(19) Europäisches Patentamt
European Patent Office
Office européen des brevets

(11) Veröffentlichungsnummer: **0 028 326**
**A1**

(12) # EUROPÄISCHE PATENTANMELDUNG

(21) Anmeldenummer: **80106195.3**

(22) Anmeldetag: **11.10.80**

(51) Int. Cl.$^3$: **C 07 C 69/716**
C 07 C 67/28, C 07 D 209/28
//A61K31/405

(30) Priorität: **25.10.79 DE 2943126**

(43) Veröffentlichungstag der Anmeldung:
**13.05.81 Patentblatt 81/19**

(84) Benannte Vertragsstaaten:
**AT BE CH DE FR GB IT LI NL**

(71) Anmelder: **Troponwerke GmbH & Co. KG**
**Berliner Strasse 156**
**D-5000 Köln 80(DE)**

(72) Erfinder: **Boltze, Karl-Heinz, Dr.**
**Am Burgtor 23**
**D-5060 Bergisch-Gladbach 3(DE)**

(72) Erfinder: **Raddatz, Siegfried, Dr.**
**Jakob-Boehme-Strasse 21**
**D-5000 Köln 80(DE)**

(72) Erfinder: **Seidel, Peter-Rudolf, Dr.**
**Alte Heide 50**
**D-5000 Köln 90(DE)**

(74) Vertreter: **Senftl, Hannes, Dr. et al,**
**c/o Bayer AG Zentralbereich Patente Marken und Lizenzen**
**D-5090 Leverkusen 1, Bayerwerk(DE)**

(54) **Lävulinoyloxyessigsäure und Verfahren zu ihrer Herstellung und ihre Verwendung bei der Synthese von Indolen.**

(57) Die vorliegende Erfindung betrifft die neue Lävulinoyloxyessigsäure, ein Verfahren zu ihrer Herstellung und ihre Verwendung bei der Synthese von pharmakologisch wertvollen Indolderivaten.

**EP 0 028 326 A1**

Troponwerke GmbH & Co. KG

KS/bc/c

Lävulinoyloxyessigsäure und Verfahren zu ihrer Herstellung und ihre Verwendung bei der Synthese von Indolen

Die vorliegende Erfindung betrifft die neue Lävulinoyloxyessigsäure, ein Verfahren zu ihrer Herstellung und ihre Verwendung bei der Synthese von pharmakologisch wertvollen Indolderivaten.

Es ist bereits bekannt, daß man Lävulinoyloxyessigsäurebenzylester als Ausgangsprodukte bei der Herstellung von Indolderivaten verwendet (vgl. DT-OS 2 234 651 und Tatsuo Yamanaka et al, Scientific Research Institute, Ltd., Japan 272 (58), Jan. 23). Die entsprechende nicht veresterte Lävulinoyloxyessigsäure ist bisher noch nicht beschrieben worden.

Bei der Herstellung der 3-Indol-acetoxyessigsäurederivate gemäß DT-OS 2 234 651 war es bisher notwendig, nach dem Indolringschluß den Benzylester, z.B. durch katalytische Hydrierung, in die freie Säure zu überführen. Durch diesen notwendigen zusätzlichen Reaktionsschritt ergab sich einmal eine

Verminderung der Ausbeute und zum anderen entstanden bei der katalytischen Hydrierung unerwünschte Nebenprodukte, insbesondere Des-Chlorverbindungen, die sich nur schwer von dem gewünschten Endprodukt abtrennen ließen.

Überraschenderweise können diese Schwierigkeiten vermieden werden, wenn man anstelle des bekannten Lävulinoyloxyessigsäurebenzylesters die neue Lävulinoyloxyessigsäure direkt einsetzt. Bei Kenntnis des Standes der Technik hätte man erwarten müssen, daß der Einsatz der erfindungsgemäßen Verbindung mit ungeschützter Carboxylgruppe, aufgrund der Reaktivität dieser Carboxylgruppe, zu unerwünschten Nebenprodukten führt. Überraschenderweise erhält man jedoch die pharmakologisch wertvollen 3-Indolacetoxyessigsäurederivate in hohen Ausbeuten und großer Reinheit.

Die Herstellung der erfindungsgemäßen neuen Lävulinoyloxyessigsäure erfolgt nach an sich bekannten Methoden durch katalytische Hydrierung des bekannten Lävulinoyloxyessigsäurebenzylesters gemäß folgendem Formelschema:

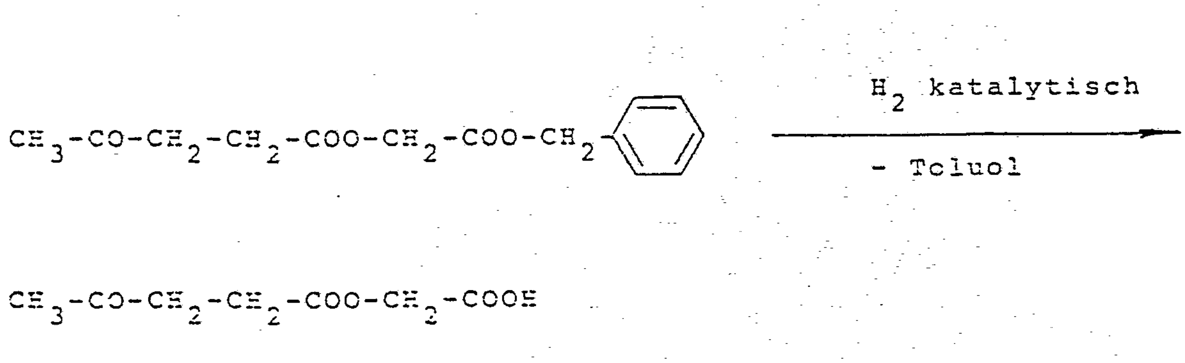

Die Umsetzung wird vorzugsweise durchgeführt in inerten organischen Lösungsmitteln unter Verwendung von Hydrierungskatalysatoren bei Temperaturen zwischen 20 und 40°C.

Als inerte organische Lösungsmittel seien vorzugsweise genannt Aceton, Essigester, Tetrahydrofuran, Dioxan, Toluol und Eisessig.

Als Hydrierungskatalysatoren seien vorzugsweise genannt Palladium/Tierkohle und $PtO_2$.

Die Umsetzung der erfindungsgemäßen Verbindung zu dem pharmakologisch wirksamen Indolderivat erfolgt beispielsweise gemäß folgendem Reaktionsschema:

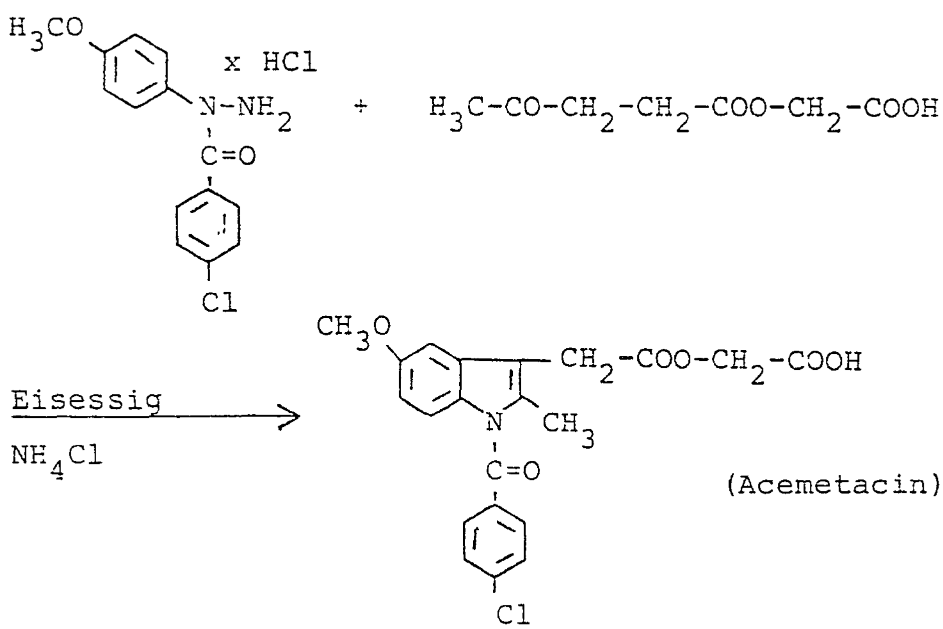


(Acemetacin)

Herstellungsbeispiele

Beispiel 1

3,0 Mol Lävulinoyloxyessigsäurebenzylester werden in 1500 ml Aceton aufgelöst und mit 25 g 5 %igem Palladium auf Aktivkohle als Katalysator versetzt und bei 20°C und 1053 mbar hydriert. Nach 165 Minuten ist die errechnete Wasserstoffmenge aufgenommen und die Hydrierung wird beendet. Der Katalysator wird abfiltriert und das Lösungsmittel bei Temperaturen unter 40°C im Vakuum abgedampft. Der ölige Rückstand wird mit 200 ml Diethylether aufgenommen und anschließend mit 1600 ml Diisopropylether versetzt. Bei 5°C bilden sich farblose Kristalle, die bei 54,5 bis 55°C schmelzen. Ausbeute: 90 % der Theorie.

Beispiel 2

Analog Beispiel 1 wird der Lävulinoyloxyessigsäurebenzylester in Essigester als Lösungsmittel hydriert. Man erhält die freie Säure in einer Ausbeute von 86 % der Theorie.

Patentansprüche

1) Lävulinoyloxyessigsäure.

2) Verfahren zur Herstellung von Lävulinoyloxyessigsäure, dadurch gekennzeichnet, daß man den Lävulinoyloxyessigsäurebenzylester katalytisch hydriert.

3) Verfahren gemäß Anspruch 2, dadurch gekennzeichnet, daß man die katalytische Hydrierung in inerten organischen Lösungsmitteln, unter Verwendung von Hydrierungskatalysatoren bei Temperaturen zwischen 20 und 40°C durchführt.

4) Verwendung von Lävulinoyloxyessigsäure bei der Herstellung von [1-(4-Chlorbenzoyl)-5-methoxy-2-methyl-3-indol]-acetoxyessigsäure.

0028326

Nummer der Anmeldung

Europäisches Patentamt

# EUROPÄISCHER RECHERCHENBERICHT

EP 80 10 6195.3

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| X | DE - A1 - 2 740 836 (TROPONWERKE)<br>* Seite 26, Zeilen 2 bis 3; Seite 32, Zeilen 5 bis 6; Seite 62, Zeilen 5 bis 6; Seite 74, Zeilen 17 bis 18 *<br>-- | 1 | C 07 C 69/716<br>C 07 C 67/28<br>C 07 D 209/28<br>//A 61 K 31/405 |
| | DE - A1 - 2 740 852 (TROPONWERKE)<br>* Seite 9, Zeilen 4 bis 5 *<br>-- | 4 | |
| D | DE - A - 2 234 651 (TROPONWERKE)<br>* Anspruch 2 *<br>---- | 2 | RECHERCHIERTE SACHGEBIETE (Int. Cl.3)<br>C 07 C 51/00<br>C 07 C 67/28<br>C 07 C 69/716<br>C 07 D 209/28 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Berlin | 04-02-1981 | KNAACK |